# EUROPÄISCHE PATENTSCHRIFT

(11) **EP 0 432 560 B1**
(45) Veröffentlichungstag und Bekanntmachung des Hinweises auf die Patenterteilung: **10.03.1993**
(21) Anmeldenummer: 90122580.5
(22) Anmeldetag: 27.11.1990
(51) Int. Cl.: A61B 19/00

(54) **Instrumentensatz zum Verschliessen von Hohlorganen und Wunden**
Set of instruments to close hollow organs and wounds
Jeu d'instruments pour fermer des organes creux et des blessures

(30) Priorität: 13.12.1989 DE 3941108
(43) Veröffentlichungstag der Anmeldung: 19.06.1991
(73) Patentinhaber: Richard Wolf GmbH, 75438 Knittlingen (DE)
(72) Erfinder: Buess, Gerhard, Prof.Dr., W-7400 Tübingen (DE); Melzer, Andreas, W-6200 Wiesbaden (DE); Gutt, Carsten N., W-6500 Mainz (DE)
(74) Vertreter: Wilcken, Thomas, Dipl.-Ing.

(56) Entgegenhaltungen:
- WO-A-89/10094
- DE-A- 2 506 471
- DE-A- 3 504 292
- DE-A- 3 709 706
- DE-A- 3 936 811
- GB-A- 2 151 142
- GB-A- 2 214 428
- US-A- 4 027 510

## Beschreibung

Die Erfindung geht aus von einem Instrumentensatz zum Verschließen von eröffneten Hohlorganen und Wunden, bestehend aus einem Außentubus und einem unlösbar mit diesem festgelegten Innentubus sowie Kanälen für hindurchzuführende Zangen zum Festhalten des Hohlorgans oder der Wundränder und aus einer durch den Innentubus hindurchzuführenden Arbeitsoptik mit Kanal zur Hindurchführung von Hilfsinstrumenten.

Ein solcher Instrumentensatz ist in der DE-PS 35 04 292 beschrieben. Dieser Instrumentensatz zur perkutanen Gallensteinentfernung besteht aus einem festlegbaren Außentubus mit Kanälen für durchzuführende Zangen zum Festhalten der Gallenblase und aus einem Innentubus für eine durchzuführende Arbeitsoptik und zur Hindurchführung von Instrumenten für die Entfernung von Gallensteinen.

Durch die Anordnung der Zangenkanäle auf der Trokarhülse als Außentubus ist nur eine ungenügende Reinigung, Desinfektion und Sterilisation möglich, und es besteht keine Möglichkeit, den zu ergreifenden Gewebeteil der Gallenblase einwandfrei sicher zu halten und entsprechend den Anforderungen manipulieren und die verhältnismäßig groß vorzunehmende Inzision der Gallenblase nach der Entfernung der Gallensteine wieder einwandfrei dicht verschließen zu können.

Des weiteren ist bekannt, Wunden mit Klammern, die mit entsprechenden Klammergeräten appliziert werden, zu verschließen, und es sind auch solche Klammergeräte für den Einsatz bei endoskopischen Eingriffen bekannt. In der DE-OS 37 13 830 ist ein solches Klammergerät für den Wundverschluß mit Klammern beschrieben, das durch den Instrumentenkanal eines Laparoskopes einführbar ist. Bei diesem Klammergerät ist die Biegebewegung wenigstens eines der Biegeglieder längs des Schaftes gerichtet, und die Druckelemente der Biegeglieder sind seitlich zueinander versetzt. Hierdurch wird eine kleine Bauweise diese Klammergerätes erreicht, so daß es bei einem Laparoskop einsetzbar ist.

Die Aufgabe der Erfindung besteht darin, einem Instrumentensatz der einleitend angeführten Art zu verbessern, um unter Vermeidung der genannten Nachteile vor allen Dingen einen sicheren Verschluß von eröffneten Hohlorganen oder Wunden, wie z. B. der inzisierten Gallenblase, zu erreichen, ohne daß ein weiterer Einstich in die Körperhöhle für die Einführung eines entsprechenden Verschließinstrumentes vorgenommen werden muß.

Diese Aufgabe wird durch die Merkmale des Anspruches 1 gelöst. Bevorzugte Ausgestaltungsmerkmale der erfindungsgemäßen Lösung sind in den Unteransprüchen angeführt.

Durch die Lösung nach der Erfindung ist es möglich, das Gewebe des Hohlorganes oder der Wunden nach Einführen des Instrumentes aus Außentubus und Innentubus mit Festhaltezangen unter Beobachtung mittels einer Arbeitsoptik durch die Bauchdecke hindurch in die Körperhöhle an das Hohlorgan heranzuführen und das Gewebe des Hohlorgans an zwei, vorteilhaft an vier sich diagonal gegenüberliegenden Stellen durch die Zangen sicher zu erfassen und festzuhalten. Sodann kann die Inzision mittels einer Aspirationssonde mit Punktionsnadel durchgeführt und die Inzision durch Dilatatoren aufgeweitet und anschließend durch den Clipapplikator verschlossen werden. Durch die Ausbildung des Instrumentensatzes gemäß der Erfindung ist eine einwandfreie Reinigung, Desinfektion und Sterilisation aller seiner Teile möglich. Das sicher erfaßte Gewebe der Gallenblase kann einfach manipuliert, und nach dem Entfernen von Gallen steinen kann der Einschnitt in der Gallenblase mittels der Inzisionsnadel wieder mittels eines Applikators und eines Clips sicher verschlossen werden.

Die Erfindung ist nachstehend im einzelnen anhand der Zeichnung erläutert. Es zeigen:
Figur 1 eine Seitenansicht des Außentubus mit Innentubus und Festhaltezangen,
Figur 2a + 2b in Seitenansicht das vergrößerte Ende eines Clipapplikators mit geöffnetem und geschlossenem Maul,
Figur 2c den gleichen Clipapplikator mit distalwärts vorgeschobenem Außenschaft,
Figur 3 einen Clip, gesehen in Richtung gegen dessen offenes Ende,
Figur 4 eine Stirnansicht gegen das distale geschlossene Ende des Applikatormaules,
Figur 5a + 5b einen ersten Dilatator in zwei um 90° versetzten, vergrößerten Seitenansichten,
Figur 6 die Seitenansicht eines zweiten Dilatators,
Figur 7 eine Seitenansicht einer üblichen Beobachtungsoptik,
Figur 8 eine Seitenansicht einer bekannten Arbeitsoptik und
Figur 9 eine Seitenansicht einer Aspirationssonde mit Punktionsnadel und ankuppelbarem, umschaltbarem Spül- und Sauganschluß.

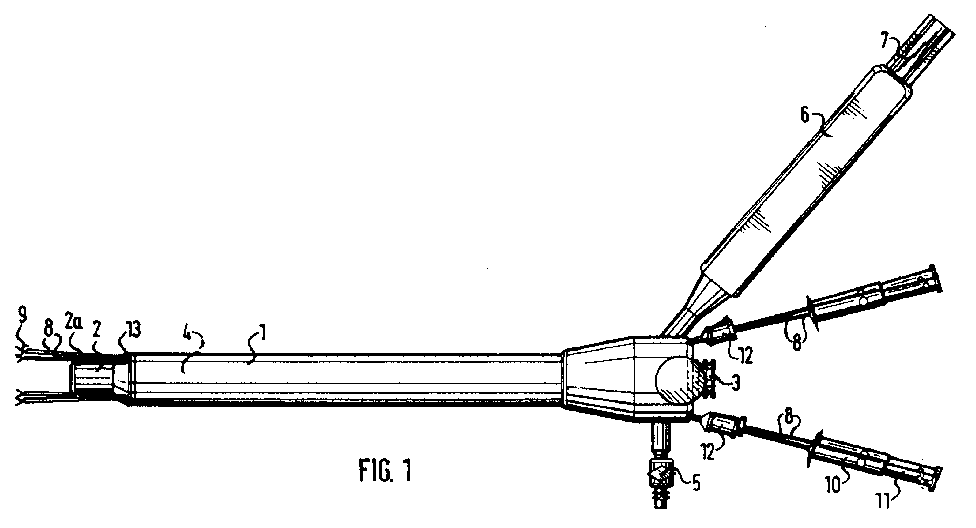

Der dargestellte Instrumentensatz besteht nach Figur 1 aus einem Außentubus 1 mit darin unlösbar festgelegtem Innentubus 2, der distale Öffnungen 2a zur Kontrolle der korrekten Lage des über den Innentubus 2 zu ziehenden Gewebes aufweist, distal gegenüber dem Außentubus vorspringt und der einen proximal durch eine Dichtung 3 abgedichteten Kanal 4 aufweist. Durch einen verschließbaren Anschlußstutzen 5 besteht die Möglichkeit über den Kanal 4 in eine Körperhöhle Gas oder eine Spülflüssigkeit einzuführen und letztere mit etwaigen Sekreten oder dergleichen abzusaugen. Der Außentubus 1 ist proximal mit einer Handhabe 6 versehen, deren starre Verlängerung 7 an einer feststehenden Vorrichtung, z. B. mittels eines verriegelbaren Gelenkarmes oder dergleichen an einem Operationstisch, befestigt werden kann, um den Tubus bzw. das gesamte Instrument für den Eingriff ortsfest halten zu können.

Zwischen Außen- und Innentubus verlaufen zwei, vorteilhaft vier sich im Rechteck diagonal gegenüberliegende Kanäle, durch die halbstarr ausgebildete Zangen 8 zum Festhalten der Gallenblase verlaufen, deren vorzugsweise beide hakenförmig ausgebildete, in einer Nut geführte Maulschenkel 9 durch proximale, federvorgespannte Handgriffteile 10 und 11 betätigbar sind. Die Zangen 8 durchlaufen proximale Dichtungen 12 der Kanäle 13 und verlaufen an ihren distalen und den proximalen Enden im Winkel zur Längsachse des Tubus 1 nach außen, um einerseits das Gewebe bezüglich seiner Lage relative zum Maulteil des Applikators manipulieren zu können und um andererseits die Handhabung zu erleichtern.

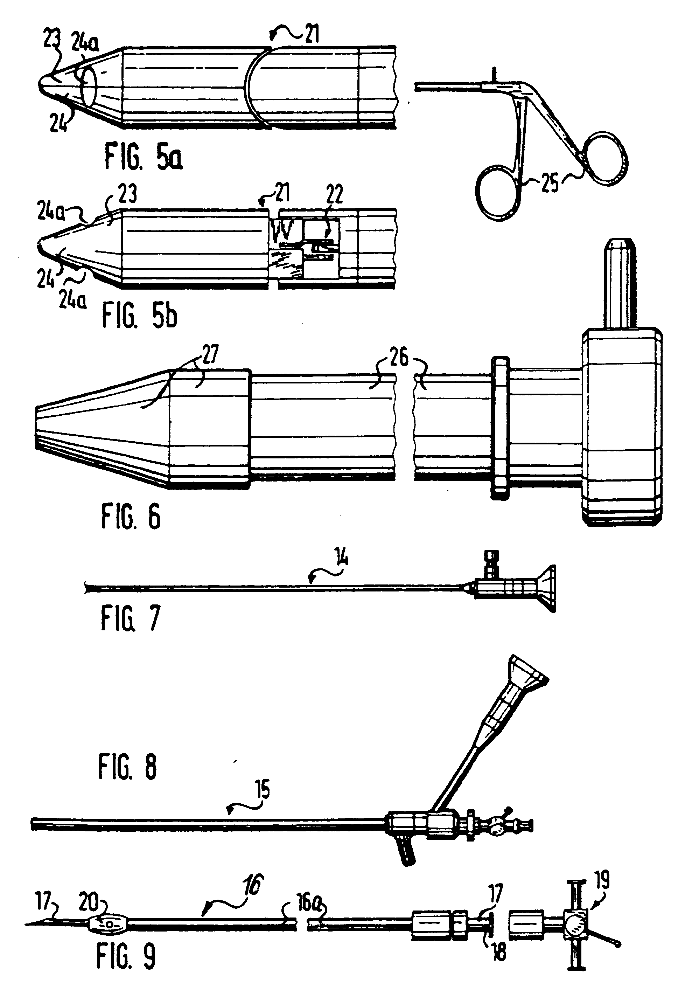

Der Instrumentensatz besteht weiter aus einer bekannten Beobachtungsoptik 14 (Fig. 7) und einer Arbeitsoptik 15 (Fig. 8) mit durchlaufendem Kanal für durchzuführende Instrumente und weiter aus einer Aspirationssonde 16 nach Fig. 9, durch deren Schaft eine ankuppelbare Punktionsnadel 17 mit proximalem Schlauchanschluß 18 geführt ist und die proximal über einen umschaltbaren Anschluß 19 an eine Zufuhr und Abfuhr einer Spülflüssigkeit anschließbar ist.

Das Sondenrohr 16a ist distal mit einer Führungsverdickung 20 versehen.

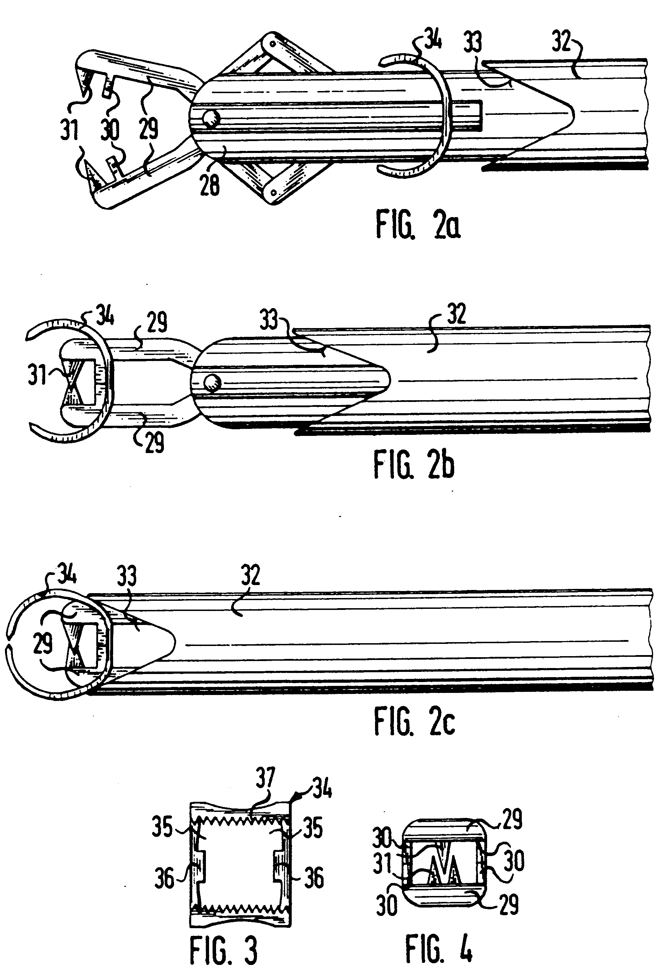

Der Instrumentensatz besteht weiter aus zwei Dilatatoren gemäß den Figuren 5a, 5b und 6 und aus einem Clipapplikator nach den Figuren 2 und 4.

Der erste Dilatator 21 nach Fig. 5a und 5b besteht aus zwei durch ein Gelenk 22 verbundenen Kegelhälften 23 und 24, die durch einen Scherengriff 25 spreizbar sind. Der zweite Dilatator 26 weist einen starren Kegel 27 auf und ist mit einem durchlaufenden Kanal für eine Beobachtungsoptik 14 nach Fig. 7 versehen, die mit dem Dilatator zusammengekuppelt werden kann.

Der Clipapplikator nach Figur 2 bis 4 besteht aus einem Schaft 28, in dessen distalem Ende die beiden Maulschenkel 29 durch eine proximale, nicht dargestellte Handhabe verschwenkt werden. Die Maulschenkel 29 werden in der Schließlage parallel im Abstand durch seitliche Anschlagpaare 30 gehalten und sind mit zueinandergekehrten Zähnen 31 versehen. Auf dem Schaft 28 ist ein Außenschaft 32 mit distalem V-förmigen Ausschnitt 33 längsverschiebbar gelagert, durch die ein deformierbarer, auf dem Schaft 28 bis hinter das Maulgelenk aufgeschobener Clip 34 in eine Applikationsstellung nach Fig. 2b und schließlich zum Schließen des dilatierten Gallenblaseneinschnittes plastisch verform wird, wie noch näher erläutert wird.

Der Clip 34 ist mit einer Durchbrechung 35 versehen, durch den der Applikator zum Übergreifen der beiden Anschläge mit leicht geöffnetem Maul hindurchschiebbar ist, bis er eine Lage nach Fig. 2a erreicht hat. Der Clip ist weiter mit nach innen gerichteten Vorsprüngen 36 versehen, die beim distalwärtigen Vorschub des Clips durch den Außenschaft 32 in die Applikationsstellung nach Fig. 2b gegen die Anschläge 30 der Maulschenkel 29 zur Anlage kommen. Die beiden Clipschenkel 37 sind auf den einander zugekehrten Innenseiten verzahnt.

Die Arbeitsweise des vorbeschriebenen Instrumentensatzes nach der Erfindung ist folgende.

Durch die Bauchdecke wird das Instrument nach Figur 1 zusammen mit der Arbeitsoptik nach Fig. 8 und der Aspirationssonde nach Fig. 9 in die Körperhöhle eingeführt, und es wird dann die Gallenblase unter Beobachtung mittels der vier Zangen 8 an vier im Rechteck liegenden Stellen erfaßt und festgehalten. Sodann wird die Gallenblase durch die Punktionsnadel 17 inzisiert und eine bestimmte Menge Gallensaft abgesaugt. Nach dem vollständigen Einführen des distalen Sondenendes 20 des Spül- und Saugrohres 16a in die Gallenblase und nach Entfernen der Punktionsnadel 17 erfolgt ein Spülen der Gallenblase.

Anschließend erfolgt das Herausziehen des Sondenrohres 16a aus der Gallenblase und aus dem Kanal der Arbeitsoptik 15, und die Sonde wird durch den ersten Dilatator 21 nach Fig. 5a und 5b ersetzt, durch die die durch die Punktionsnadel 17 erfolgte Inzision der Gallenblase mittels des zu spreizenden Dilatatorkegels 23,24 aufgeweitet wird. Anschließend wird der Dilatator 21 und die Arbeitsoptik 15 durch den zweiten Dilatator 26 mit der durch ihn hindurchgeführten Beobachtungsoptik 14 nach Fig. 7 ersetzt und die Inzision der Gallenblase weiter aufgeweitet, indem die durch die Zangen 8 erfaßte Gallenblase auf den Innentubus 2 aufgezogen wird. Die Zangen 8 sind dabei so um ihre Längsachse verdreht, daß die erfaßten Falten der Gallenblase eine Ebene parallel oder etwa parallel zum Umfang des Dilatators einnehmen.

Im Anschluß daran wird der zweite Dilatator 26 mit der Beobachtungsoptik 14 entfernt und durch die Arbeitsoptik 15 ersetzt, durch die hindurch nunmehr die Zertrümmerung und/oder Entfernung von Gallensteinen erfolgt.

Es wird anschließend der Clipapplikator nach Fig. 2a durch die aus dem Tubus 1,2 herausgenommene Arbeitsoptik mit geschlossenem Maul hindurchgeführt, worauf der Clip 34 durch den Außenschaft 32 in die Applikationsstellung nach Fig. 2b distalwärts geschoben wird. Anschließend ist der Außenschaft 32 weiter distalwärts zu schieben, so daß nunmehr die Schenkel des freigewordenen Clips 34 durch den V-förmigen Einschnitt 33 deformierend das Blasengewebe mittels der Clipverzahnung erfassen und zusammendrücken, wodurch nunmehr die Inzision einwandfrei verschlossen ist. Schließlich wird der Clipapplikator nach Zurückziehen des verschiebbaren Außenschftes 32 geöffnet und mit der Arbeitsoptik herausgezogen, und sodann wird der Tubus 1, 2 ebenfalls aus der Körperhöhle entfernt.

## Patentansprüche

1. Instrumentensatz zum Verschließen von eröffneten Hohlorganen und Wunden, bestehend aus einem Außentubus (1) und einem unlösbar mit diesem festgelegten Innentubus (2) sowie Kanälen (13) für hindurchzuführende Zangen zum Festhalten des Hohlorgans oder der Wundränder und aus einer durch den Innentubus (2) hindurchzuführenden Arbeitsoptik (14) mit Kanal zur Hindurchführung von Hilfsinstrumenten und dergleichen, dadurch gekennzeichnet, daß zwischen dem Außentubus (1) und dem distal über den Außentubus vorragenden Innentubus (2) mindestens zwei Kanäle (13) für Haltezangen (8) verlaufen, daß eine durch den Innentubus (2) einführbare Beobachtungsoptik (14) und eine Aspirationssonde (16) mit herausnehmbarer Punktionsnadel (17) vorgesehen sind, daß Dilatatoren (21,26) zum Aufweiten einer Inzision in einem Hohlorgan oder der Wunde gegen die Sonde (16) austauschbar sind und daß die Beobachtungsoptik (14) gegen die Arbeitsoptik (15) austauschbar vorgesehen ist, die im Innentubus (2) lösbar fixierbar und mit einem Clipapplikator (28-33) mit einem auf ihn distal aufschiebbaren und distalwärts abgebbaren Clip (34) zum Verschließen eines eröffneten Hohlorgans oder einer Wunde versehen ist.

2. Instrumentensatz nach Anspruch 1, dadurch gekennzeichnet, daß die zwischen Außen- und Innentubus (1,2) verlaufenden Kanäle (13) proximal nach außen abgewinkelt sind und eine Dichtung (12) für die proximal betätigbaren, halbstarr ausgebildeten Haltezangen (8) aufweisen, deren jeweils in einer Nut geführten beiden distalen Maulteilbacken (9) hakenförmig ausgebildet sind.

3. Instrumentensatz nach Anspruch 1 oder 2, dadurch gekennzeichnet, daß eine Ebene, die quer zur Ebene des Maules (9) der Haltezangen (8) verläuft, mit der Längsachse der Zangen einen Winkel bildet.

4. Instrumentensatz nach einem der Ansprüche 1 bis 3, dadurch gekennzeichnet, daß der durch den Innentubus (2) gebildete Kanal proximal durch eine Dichtung (3) abgedichtet und mit einem abschließbaren Hahn (5) zur Zu- und Abfuhr von Spülflüssigkeit oder Körperhöhlengas versehen ist.

5. Instrumentensatz nach einem der Ansprüche 1 bis 4, dadurch gekennzeichnet, daß der Außentubus (1) mit einer Handhabe (6) versehen ist, deren starre Verlängerung (7) festlegbar ist.

6. Instrumentensatz nach einem der Ansprüche 1 bis 5, dadurch gekennzeichnet, daß ein erster Dilatator (21) für die Aufweitung der vorgenommenen Inzision mittels der Punktionsnadel (17) aus einem in Achsrichtung geteilten Kegel (23,24) besteht, dessen gelenkig verbundene Hälften durch eine proximale Handhabe (25) spreizbar sind.

7. Instrumentensatz nach Anspruch 6, dadurch gekennzeichnet, daß die beiden Kegelhälften (23, 24) beidseitig mit sich gegenüberliegenden Vertiefungen (24a) versehen sind, in die die Wundkanten des eröffneten Organs oder die Wundränder der Wunde eingreifen.

8. Instrumentensatz nach Anspruch 1 bis 5, dadurch gekennzeichnet, daß neben dem Dilatator (21) nach Anspruch 6 oder 7 ein zweiter Dilatator (26) mit einem starren Kegel (27) und mit einem zentralen Kanal zur Durchführung der mit dem proximalen Ende des Dilatators (26) kuppelbaren Beobachtungsoptik (14) vorgesehen ist.

9. Instrumentensatz nach Anspruch 1 und einem der Ansprüche 2 bis 8, dadurch gekennzeichnet, daß die Aspirationssonde (16) aus einer proximal mit einem Spül- und Saugrohr (16a) kuppelbaren Punktionsnadel (17) besteht und proximal mit einem umschaltbaren Schlauchanschluß (19) verbindbar ist und daß das Saugrohr (16a) am distalen Ende eine Rohrerweiterung (20) aufweist.

10. Instrumentensatz nach Anspruch 1 und einem der Ansprüche 2 bis 9, dadurch gekennzeichnet, daß der Clipapplikator (28 bis 33) ein durch eine proximale Handhabe verschließbares Maul (29 bis 31) besitzt, dessen Maulschenkel (29) mit einander zugekehrten Greifzähnen (31) und jeweils beidseitigen Anschlagpaaren (30) für die Anlage der beiden Vorsprünge (36) des Clips (34) versehen sind und das proximalseitig des Zangenmaules ein in zwei gegenüberliegenden Nuten mit proximalwärts abnehmender Tiefe geführter, plastisch verformbarer Clip (34) aufschiebbar und lösbar festlegbar ist, der durch einen auf dem Applikator verschiebbaren Außenschaft (32) zum Verschließen des eröffneten Hohlorgans oder der Wunde verformbar und im Anschluß daran nach erfolgtem Öffnen der beiden Maulschenkel (29) durch den Applikator freigebbar ist.

11. Instrumentensatz nach Anspruch 10, dadurch gekennzeichnet, daß der vom Applikator (28 bis 33) durchgreifbare Clip (34) eine Ausnehmung (35) aufweist, die mit zwei gegenüberliegenden, gegen die Anschlagpaare (30) der Maulschenkel (29) durch Verschieben des Clips mittels dem Außenschaft (32) zur Anlage kommenden Vorsprüngen (36) versehen ist.

12. Instrumentensatz nach Anspruch 10 oder 11, dadurch gekennzeichnet, daß der auf dem Applikator (28 bis 33) längsverschiebbare Außenschaft (32) distal beidseitig mit einen V-förmigen Ausschnitt (33) versehen ist.

## Claims

1. A set of instruments for the closing of opened hollow organs and wounds, consisting of an outer tube (1) and of an inner tube (2), fixed undetachably therewith, and also of channels (13) for forceps, which are to be passed therethrough, to secure the hollow organ or the edges of the wound, and of a working optical system (14), to be passed through the inner tube (2), with a channel for the passage through of auxiliary instruments and the like, characterised in that at least two channels (13) for holding forceps (8) run between the outer tube (1) and the inner tube (2), projecting distally beyond the outer tube, that an observation optical system (14), insertable through the inner tube (2), and an aspiration probe (16) with removable puncture needle (17) are provided, that dilators (21, 26) for widening an incision in a hollow organ or the wound are exchangeable for the probe (16) and that the observation optical system (14) is provided so as to be exchangeable for the working optical system (15), which can be releasably secured in the inner tube (2) and is provided with a clip applicator (28-33) with a clip (34), which can be pushed thereon distally and is releasable in a distal direction, to close an opened hollow organ or a wound.

2. A set of instruments according to claim 1, characterised in that the channels (13) running between the outer- and inner tube (1,2) are bent proximally outwards and have a seal (12) for the holding forceps (8), which are actuable proximally and are made semi-rigid, the two distal jaw piece grips (9) of which, guided in each case in a groove, are constructed in a hooked shape.

3. A set of instruments according to claim 1 or 2, characterised in that a plane, which extends transversely to the plane of the jaw (9) of the holding forceps (8), forms an angle with the longitudinal axis of the forceps.

4. A set of instruments according to one of claims 1 to 3, characterised in that the channel formed by the inner tube (2) is sealed proximally by a seal (3) and is provided with a closable tap (5) for the supply and removal of flushing fluid or gas from body cavities.

5. A set of instruments according to one of claims 1 to 4, characterised in that the outer tube (1) is provided with a handle (6), the rigid extension (7) of which is securable.

6. A set of instruments according to one of claims 1 to 5, characterised in that a first dilator (21) for widening the incision which has been made by means of the puncture needle (17) comprises a cone (23,24) divided in the axial direction, the articulatedly connected halves of which are spreadable by a proximal handle (25).

7. A set of instruments according to claim 6, characterised in that the two cone halves (23, 24) are provided on both sides with depressions (24a) situated opposite each other, into which the borders of the wound of the opened organ or the wound edges of the wound engage.

8. A set of instruments according to claims 1 to 5, characterised in that in addition to the dilator (21) according to claim 6 or 7, a second dilator (26) is provided, having a rigid cone (27) and a central channel to pass through the observation optical system (14), which can be coupled with the proximal end of the dilator (26).

9. A set of instruments according to claim 1 and one of claims 2 to 8, characterised in that the aspiration probe (16) comprises a puncture needle (17) which can be coupled proximally with a flushing- and suction tube (16a) and is connectable proximally with a reversible hose connection (19) and that the suction tube (16a) has a widening (20) of the tube at the distal end.

10. A set of instruments according to claim 1 and one of claims 2 to 9, characterised in that the clip applicator (28 to 33) has a jaw (29 to 31) which is closable by a proximal handle, the jaw shanks (29) of which are provided with mutually opposed gripper teeth (31), and in each case with pairs of stops (30) on both sides, to rest the two projections (36) of the clip (34) and that on the proximal side of the forceps jaw a plastically deformable clip (34), which is guided in two opposing grooves having a depth decreasing in the proximal direction, is slidable and releasably securable, which clip is deformable by an outer shaft (32), slidable on the applicator, to close the opened hollow organ or the wound, and is able to be releasable thereafter by the applicator after the two jaw shanks (29) have been opened.

11. A set of instruments according to claim 10, characterised in that the clip (34), which is penetrable by the applicator (28 to 33), has a recess (35) which is provided with two opposing projections (36), which are brought to rest against the pairs of stops (30) of the jaw shanks (29) by displacing the clip by means of the outer shaft (32).

12. A set of instruments according to claim 10 or 11, characterised in that the outer shaft (23), which is displaceable longitudinally on the applicator (28 to 33), is provided distally on both sides with a V-shaped cut-out (33).

## Revendications

1. Jeu d'instruments pour fermer des organes creux ouverts et des blessures ouvertes, constitué par un tube extérieur (1) et un tube intérieur (2) fixé de façon inamovible au précédent, ainsi que par des canaux (13) pour des pinces que l'on doit faire passer dans les canaux, pour maintenir fermement l'organe creux ou les bords de la blessure, et par une optique de travail (14) que l'on doit faire passer dans le tube intérieur (2) et qui comporte un canal permettant d'y faire passer des instruments auxiliaires et analogues, caractérisé en ce qu'au moins deux canaux (13) pour des pinces de maintien (8) s'étendent entre le tube extérieur (1) et le tube intérieur (2), qui fait saillie sur le côté distal au-delà du tube extérieur, en ce qu'il est prévu une optique d'observation (14) pouvant être introduite dans le tube intérieur (2) et une sonde d'aspiration (16) équipée d'une aiguille à ponction (17) pouvant être retirée, en ce qu'on peut remplacer la sonde (16) par des dilatateurs (21,26) destinés à élargir une incision dans un organe creux ou la blessure, et en ce qu'il est prévu de remplacer par l'optique d'observation (14) l'optique de travail (15), qui peut être fixée de façon amovible dans le tube intérieur (2) et est pourvue d'un applicateur à clip (28-33) comportant un clip (34) pouvant être emmanché sur et être retiré de l'applicateur sur le côté distal pour fermer un organe creux ouvert ou une blessure.

2. Jeu d'instruments selon la revendication 1, caractérisé en ce que les canaux (13), qui s'étendent entre le tube extérieur et le tube intérieur (1,2) sont coudés vers l'extérieur sur le côté proximal et possèdent une garniture d'étanchéité (12) pour les pinces de maintien (8) qui sont semi-rigides et peuvent être actionnées sur le côté proximal et dont les deux mâchoires distales (9), qui sont guidées chacune dans une rainure, sont réalisées en forme de crochet.

3. Jeu d'instruments selon la revendication 1 ou 2, caractérisé en ce que le plan, qui est transversal au plan de la bouche (9) des pinces de maintien (8), fait un angle avec l'axe longitudinal de la pince.

4. Jeu d'instruments selon l'une des revendications 1 à 3, caractérisé en ce que le canal formé par le tube intérieur (2) est étanchéifié sur le côté proximal par une garniture d'étanchéité (3) et est équipé d'un robinet (5), qui peut être fermé, pour l'introduction et l'évacuation d'un liquide de lavage ou d'un gaz d'une cavité corporelle.

5. Jeu d'instruments selon l'une des revendications 1 à 4, caractérisé en ce que le tube extérieur (1) est équipé d'une poignée (6), dont le prolongement rigide (7) peut être fixé.

6. Jeu d'instruments selon l'une des revendications 1 à 5, caractérisé en ce qu'un premier dilatateur (21) est destiné à élargir l'incision réalisée au moyen de l'aiguille à ponction (17) et est constitué par un cône (23,24) divisé dans la direction axiale et dont les moitiés, qui sont reliées d'une manière articulée, peuvent être écartées au moyen d'une poignée proximale (25).

7. Jeu d'instruments selon la revendication 6, caractérisé en ce que les deux moitiés (23,24) du cône sont pourvues, des deux côtés, de renfoncements opposés (24a), dans lesquels s'engagent les bords de la blessure de l'organe ouvert ou les bords de la blessure.

8. Jeu d'instruments selon les revendications 1 à 5, caractérisé en ce qu'en plus du dilatateur (21) selon la revendication 6 ou 7, il est prévu un second dilatateur (26) possédant un cône rigide (27) et un canal central permettant de faire passer l'optique d'observation (14) qui peut être accouplée à l'extrémité proximale du dilatateur (26).

9. Jeu d'instruments selon la revendication 1 et l'une des revendications 2 à 8, caractérisé en ce que la sonde d'aspiration (16) est constituée par une aiguille à ponction (17) qui peut être accouplée, sur le côté proximal, à un tube de lavage et d'aspiration (16a) et peut être raccordée, sur le côté proximal, à un raccord de tuyau commutable (19), et en ce que le tube d'aspiration (16a) possède, à l'extrémité distale, une partie élargie (20).

10. Jeu d'instruments selon l'une des revendications 1 et l'une des revendications 2 à 9, caractérisé en ce que l'applicateur à clip (28 à 33) possède une embouchure (29 à 31), qui peut être fermée par une poignée proximale et dont la mâchoire (29) est équipée de dents d'accrochage (31), qui sont tournées l'une vers l'autre, et de couples respectifs de butée (30), qui sont situées des deux côtés et contre lesquelles viennent s'appliquer les deux appendices saillants (36) du clip (34) et en ce que, sur le côté proximal de la bouche de la pince peut être emmanché et fixé de façon amovible un clip déformable plastiquement (34), qui est guidé dans deux rainures opposées possédant une profondeur qui diminue en direction du côté proximal et qui peut être déformé par une tige extérieure (32) déplaçable sur l'applicateur, pour fermer l'organe creux ouvert ou la blessure et peut être ensuite libéré par l'applicateur, après l'ouverture des deux mâchoires (29).

11. Jeu d'instruments selon la revendication 10, caractérisé en ce que le clip (34), qui peut être saisi par l'applicateur (28 à 33), comporte un évidement (35), qui est pourvu de deux appendices saillants opposés (36) qui viennent s'appliquer contre les couples de butées (30) des mâchoires (29) de la bouche des pinces, sous l'effet du déplacement du clip au moyen de la tige extérieure (32).

12. Jeu d'instruments selon la revendication 10 ou 11, caractérisé en ce que la tige extérieure (32), qui est déplaçable longitudinalement sur l'applicateur (28 à 33), comporte sur le côté distal, sur chacune de ses faces, une découpe en forme de V (33).
